# EUROPEAN PATENT SPECIFICATION

(11) **EP 3 919 109 B1**
(45) Date of publication and mention of the grant of the patent: **25.06.2025**
(21) Application number: 20748168.0
(22) Date of filing: 23.01.2020
(51) Int. Cl.: A61B 5/0215, A61B 5/00, A61M 25/00, A61M 60/139, A61M 60/295, A61M 60/531, A61M 60/816, A61M 60/841, A61M 60/843

(54) **INTRA-AORTIC BALLOON CATHETER**
INTRAAORTALER BALLONKATHETER
CATHÉTER À BALLONNET INTRA-AORTIQUE

(30) Priority: 30.01.2019 JP 2019014277
(43) Date of publication of application: 08.12.2021
(73) Proprietor: Zeon Corporation, Tokyo 100-8246 (JP)
(72) Inventor: FUJIHATA,Takashi, Tokyo 100-8246 (JP); ISHIDA, Takaki, Tokyo 100-8246 (JP)
(74) Representative: Maiwald GmbH
(86) International application number: PCT/JP2020/002336
(87) International publication number: WO 2020/158572

(56) References cited:
- WO-A1-2018/180976
- WO-A1-2019/013201
- CN-A- 105 852 833
- JP-A- 2016 093 289
- JP-A- 2016 093 290
- JP-A- 2016 190 011
- JP-A- 2017 176 719
- US-A1- 2015 141 854
- US-B1- 6 398 738

## Description

### TECHNICAL FIELD

The present invention relates to an intra-aortic balloon catheter.

### BACKGROUND ART

As therapy for cardiac dysfunction, there is known an intra-aortic balloon pumping method (IABP method) in which a balloon catheter is inserted into an aorta and a balloon is expanded and contracted in accordance with a heartbeat to assist a cardiac function.

As the intra-aortic balloon catheter used in the IABP method, there is suggested a sensor-attached balloon catheter in which a sensor that detects a pressure by using light is attached to a distal end portion of the balloon catheter, and a signal of a detected blood pressure is transmitted to a proximal end of the balloon catheter through an optical fiber (for example, refer to Patent Document 1).

The catheter described in Patent Document 1 includes a distal tip, and a sensor accommodating hole in which a sensor is disposed and a through-hole through which an optical fiber connected to the sensor is inserted are formed inside the distal tip. For example, the optical fiber is fixed to the inside of the through-hole by means such as bonding by the following method.

That is, the optical fiber in which a sensor is connected to a distal end is inserted into the through-hole, and a distal side thereof is drawn to an outer side of the sensor accommodating hole. Then, a sensor accommodating hole is filled with adhesive, and the distal side of the optical fiber is inserted into the through-hole. At this time, since the distal side of the optical fiber passes through the adhesive filled in the sensor accommodating hole, the adhesive adheres to the periphery of the optical fiber, and the adhesive is inserted into the through-hole in combination with the optical fiber. Accordingly, the through-hole is filled with the adhesive, and the optical fiber can be fixed to the inside of the through-hole by the adhesive.

Patent Document 2 discloses a catheter with one or more filling holes to insert an adhesive into a through hole of the catheter. An optical fiber is also provided in the through hole, and the adhesive is used to fasten a ring attached to the optical fiber.

Patent Document 3 discloses a catheter with an optical fiber in a through hole of the catheter. Only one filling hole for adhesive is provided in the catheter.

### CITATION LIST

### PATENT DOCUMENTS

Patent Document 1: JP 2010-233883 A
Patent Document 2: JP 2016-190011 A
Patent Document 3: WO 2019/013201 A1

### SUMMARY OF THE INVENTION

### PROBLEM TO BE SOLVED BY THE INVENTION

However, in the method of fixing the optical fiber as disclosed in the related art, since the sensor accommodating hole is filled with the adhesive, the adhesive adheres to an inner wall of the sensor accommodating hole. In a case where the adhesive adheres to the sensor disposed inside the sensor accommodating hole, there is a concern that accuracy of the sensor decreases, and measurement accuracy of fluctuations in a blood pressure inside an aorta decreases.

The invention has been made in consideration of such circumstances, and an object thereof is to provide an intra-aortic balloon catheter capable of measuring fluctuations in blood pressure inside an aorta with high accuracy.

### MEANS FOR SOLVING PROBLEM

To accomplish the above-described object, according to an aspect of the invention, there is provided an intra-aortic balloon catheter including:
a sensor capable of measuring a pressure by using light;
an optical fiber connected to the sensor; and
a distal tip having a sensor accommodating hole accommodating the sensor and a through-hole connected to the sensor accommodating hole to pass the optical fiber through,
in which a filling-hole in which one end is opened from an outer peripheral surface of the distal tip and the other end is connected to the through-hole is formed in the distal tip in a manner capable of filling the inside of the through-hole with a resin.

In the intra-aortic balloon catheter according to the invention, the filling-hole in which one end is opened from the outer peripheral surface of the distal tip and the other end is connected to the through-hole is formed in the distal tip in a manner capable of filling the inside of the through-hole with a resin. Accordingly, the through-hole can be directly filled with a resin through the filling-hole, and the optical fiber can be fixed to the inside of the through-hole with the resin. That is, in the intra-aortic balloon catheter according to the invention, it is not necessary to fill a sensor accommodating hole with a resin unlike the method of fixing an optical fiber disclosed in the related art. Accordingly, there is no concern that the resin adheres to the sensor disposed in the sensor accommodating hole, deterioration of accuracy of the sensor due to adhesion of the resin is prevented, and fluctuations in a blood pressure inside an aorta can be measured with high accuracy.

In addition, since the inside of the through-hole is directly filled with the resin, a void is less likely to occur in a resin layer formed in the through-hole. Accordingly, the resin is prevented from being peeled off from an inner wall surface of the through-hole, and the optical fiber can be fixed to the inside of the through-hole with high fixing strength.

According to the invention, the inside of the through-hole and the filling-hole is filled with the resin. In this case, a sufficient amount of resin is supplied to the inside of the through-hole, and thus the fixing strength of the optical fiber fixed to the inside of the through-hole can be sufficiently enhanced.

According to the invention, the filling-hole comprises filling-holes, and each of the filling-holes is disposed at the distal tip along an axial direction of the through-hole. In this case, the inside of the through-hole can be filled with the resin through any one of the filling-holes, and a resin filling state in the through-hole can be confirmed through the other adjacent filling-holes. Accordingly, a resin filling amount can be adjusted in correspondence with the resin filling state inside the through-hole, and the inside of the through-hole can be filled with an optimal amount of resin. An inner wall of the through-hole is fixed to the optical fiber with the resin.

Preferably, a first filling-hole of the filling-holes is connected to a proximal end portion of the through-hole, and a second filling-hole of the filling-holes is connected to a distal end portion of the through-hole. With this configuration, the inside of the through-hole can be filled with the resin through the first filling-hole and the resin filling state inside the through-hole can be confirmed through the second filling-hole. In addition, according to the above-described configuration, a wide region from a proximal end portion of the through-hole to a distal end portion thereof can be easily filled with a sufficient amount of resin.

A third filling-hole of the filling-holes may be connected to the through-hole, and the third filling-hole may be located between the first filling-hole and the second filling-hole. With this configuration, the inside of the through-hole can be filled with the resin through the first filling-hole, and the resin filling state inside the through-hole in a region between the first filling-hole and the third filling-hole can be confirmed through the third filling-hole. In addition, the inside of the through-hole can be further filled with the resin through the third filling-hole, and a resin filling state inside the through-hole in a region between the second filling-hole and the third filling-hole can be confirmed through the second filling-hole.

In addition, the filling of the inside of the through-hole with the resin and the confirmation of the filling state are performed through the two filling-holes, and thus the amount of resin filled inside the through-hole can be finely adjusted.

Preferably, the filling-hole extends along a diameter direction of the distal tip. With this configuration, an outer peripheral surface of the distal tip and the through-hole are connected at the shortest distance through the filling-hole, and thus the inside of the through-hole can be easily filled with the resin through the filling-hole.

### BRIEF DESCRIPTION OF DRAWINGS

Fig. 1 is a schematic cross-sectional view of an intra-aortic balloon catheter according to a first embodiment of the invention;
Fig. 2A is a perspective view of a distal tip of the intra-aortic balloon catheter illustrated in Fig. 1;
Fig. 2B is a perspective view of a distal tip of an intra-aortic balloon catheter according to a second embodiment of the invention;
Fig. 3A is a schematic cross-sectional view of the distal tip illustrated in Fig. 2A;
Fig. 3B is a schematic cross-sectional view of the distal tip illustrated in Fig. 2B;
Fig. 4A is a schematic cross-sectional view illustrating a process of filling a through-hole formed in the distal tip illustrated in Fig. 3A with a resin through a first filling-hole;
Fig. 4B is a schematic cross-sectional view illustrating a process of filling the through-hole illustrated in Fig. 4A with a resin through a third filling-hole;
Fig. 4C is a schematic cross-sectional view illustrating a process after filling the through-hole with the resin;
Fig. 4D is a schematic cross-sectional view illustrating a process of forming a resin film on an outer peripheral surface of a body portion; and
Fig. 4E is a schematic cross-sectional view illustrating a modification example of the resin film illustrated in Fig. 4D.

### MODE(S) FOR CARRYING OUT THE INVENTION

Hereinafter, the invention will be described on the basis of embodiment illustrated in the accompanying drawings.

### First Embodiment

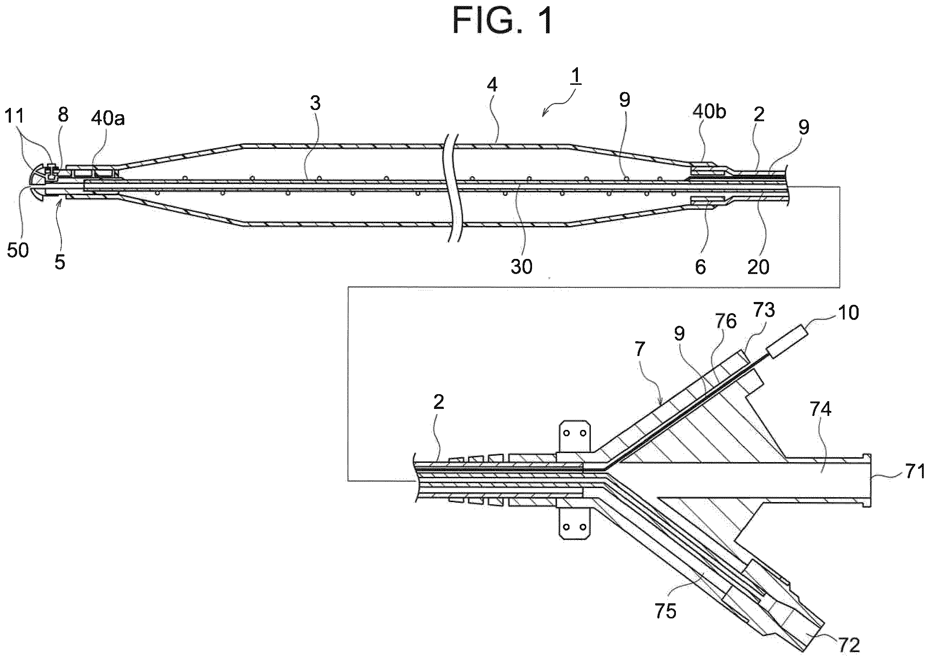

As illustrated in Fig. 1, a balloon catheter 1 according to a first embodiment of the invention is an intra-aortic balloon catheter used in an IABP method, and includes a balloon portion 4 expanding and contracting in accordance with a heartbeat. The balloon portion 4 is constituted by a thin film having a film thickness of approximately 50 to 150 µm. Although not particularly limited, a material of the thin film is preferably a material with excellent bending fatigue resistant characteristics, and examples thereof include polyurethane and the like.

An outer diameter and a length of the balloon portion 4 are determined in correspondence with an inner volume of the balloon portion 4 having a great effect on a cardiac function assisting effect, an inner diameter of an artery blood vessel, or the like. Although not particularly limited, the inner volume of the balloon portion 4 is 20 to 50 cc, the outer diameter of the balloon portion 4 is preferably 12 to 16 mm in an expanded state, and the length is preferably 150 to 250 mm.

A distal end portion 40a of the balloon portion 4 is attached to an outer periphery of a distal tip 5 by means such as thermal fusion or bonding. A wire insertion hole 50 that communicates in an axial direction is formed in the distal tip 5, and a distal end portion of an inner tube 3 enters the proximal end side thereof. The distal end portion of the inner tube 3 is connected to a proximal end portion of the distal tip 5 by means such as thermal fusion or bonding so that a wire passage 30 inside the inner tube 3 and the wire insertion hole 50 communicate with each other.

A proximal end portion 40b of the balloon portion 4 is connected to an outer periphery of a distal end portion of an outer tube 2 directly or through contrast marker 6 constituted by a radiation opaque metal ring or the like. A pressure fluid is introduced and discharged to and from the inside of the balloon portion 4 through a pressure fluid conduction path 20 formed inside the outer tube 2, and thus the balloon portion 4 expands or contracts. Connection between the balloon portion 4 and the outer tube 2 is performed by thermal fusion or bonding with adhesive.

The inner tube 3 extends in an axial direction through the inside of the balloon portion 4 and the outer tube 2, and the wire passage 30 that does not communicate with the inside of the balloon portion 4 and the pressure fluid conduction path 20 formed inside the outer tube 2 is formed inside the inner tube 3, and communicates with a secondary port 72 of a branch portion 7 to be described later.

When the intra-aortic balloon catheter 1 is inserted into the artery, the contracted balloon portion 4 is wound around the inner tube 3 located inside the balloon portion 4. The wire passage 30 is used as a lumen into which a guide wire used for conveniently inserting the balloon portion 4 into the artery is inserted.

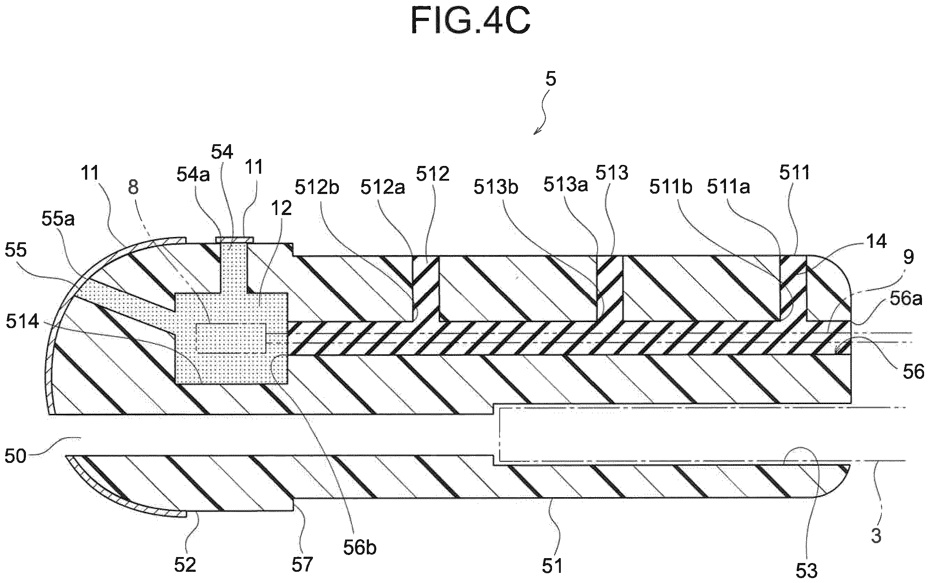

On an outer side of the inner tube 3, an optical fiber 9 extends in an axial direction of the inner tube 3. More specifically, at the inside of the outer tube 2 that extends between the branch portion 7 and the proximal end portion 40b of the balloon portion 4, the optical fiber 9 extends straightly in the axial direction along an outer side (outer peripheral surface) of the inner tube 3. In addition, at the inside of the balloon portion 4 located between the proximal end portion 40b and the distal end portion 40a of the balloon portion 4, the optical fiber 9 extends in the axial direction while being wound around the outer peripheral surface of the inner tube 3 in a spiral shape. In addition, at the inside of the distal tip 5 (body portion 51 to be described later) in which the distal end portion 40a of the balloon portion 4 is located, the optical fiber 9 extends straightly in the axial direction of the inner tube 3 (refer to Fig. 4C). Note that, the above-described balloon portion 4 in a contracted state is wound around the inner tube 3 around which the optical fiber 9 is wound in a spiral shape at the inside of the balloon portion 4.

Any portion located between a proximal end side and a distal end side of the optical fiber 9 is not fixed to an outer peripheral surface of the inner tube 3 or the like by fixing means such as adhesive, and only the proximal end side and the distal end side of the optical fiber 9 are respectively fixed to a tertiary port 73 and a pressure sensor 8.

The branch portion 7 is connected to a proximal end portion of the outer tube 2. The branch portion 7 is formed separately from the outer tube 2, and is connected to the outer tube 2 with means such as thermal fusion or bonding. A primary passage 74 in which a primary port 71 through which the pressure fluid is introduced and discharged to and from the inside of the pressure fluid conduction path 20 inside the outer tube 2 and the balloon portion 4 is formed, and a secondary passage 75 in which the secondary port 72 communicating with the wire passage 30 inside the inner tube 3 are formed in the branch portion 7.

The primary port 71 is connected to a pumping device (not illustrated), and the pressure fluid is introduced and discharged to and from the inside of the balloon portion 4 by the pumping device. The primary passage 74 linearly extends at the inside of the branch portion 7, and is connected straightly to the pressure fluid conduction path 20. Accordingly, at the inside of the pressure fluid conduction path 20, flow passage resistance of the pressure fluid introduced and discharged through the primary port 71 is reduced, and responsiveness of expansion and contraction of the balloon portion 4 can be enhanced. Although not particularly limited, as the pressure fluid, a helium gas having a small viscosity and a small mass or the like is used so that the balloon portion 4 quickly expands and contracts in correspondence with driving of the pumping device.

The tertiary port 73 is formed in the branch portion 7 in addition to the primary port 71 and the secondary port 72. A tertiary passage 76 into which the optical fiber 9 is inserted communicates with the tertiary port 73, and a proximal end side of the optical fiber 9 is drawn from the tertiary port 73. The optical fiber 9 drawn from the tertiary port 73 is bonded and fixed to the inside of the tertiary passage 76 closer to a drawing port of the tertiary port 73. The drawing port of the optical fiber 9 in the tertiary port 73 is configured so that a fluid inside the primary passage 74 and the secondary passage 75 is not leaked to the outside.

An optical connector 10 is connected to the proximal end of the optical fiber 9. The pressure sensor 8 that measures a blood pressure is connected to a distal end of the optical fiber 9 as to be described later in detail. A blood pressure measurement device (not illustrated) is connected to the optical connector 10. The pumping device is controlled in correspondence with a heartbeat on the basis of fluctuations in a blood pressure measured by the blood pressure measuring device, and the balloon portion 4 is caused to expand and contract in a short period of 0.4 to one second.

An inner peripheral surface of the outer tube 2 and an outer peripheral surface of the inner tube 3 are fixed to each other with adhesive. When the outer tube 2 and the inner tube 3 are fixed in this manner, the flow passage resistance of the pressure fluid conduction path 20 inside the outer tube 2 decreases, and thus responsiveness of the balloon portion 4 is improved. Although not particularly limited, as the adhesive used in the fixing, adhesive such as a cyanoacrylate adhesive and an epoxy adhesive can be used, and a cyanoacrylate adhesive is particularly preferably used.

In the balloon catheter 1 of this embodiment, although not particularly limited, an outer diameter of the inner tube 3 is preferably 0.5 to 1.5 mm, and the outer diameter of the inner tube 3 is preferably 30% to 60% of an inner diameter of the outer tube 2. In this embodiment, the outer diameter of the inner tube 3 is approximately the same along the axial direction. For example, the inner tube 3 is constituted by a synthetic resin tube such as polyurethane, polyvinyl chloride, polyethylene, polyamide, or polyether ether ketone (PEEK), a nickel titanium alloy thin tube, a stainless steel thin tube, or the like. In addition, in a case where the inner tube 3 is constituted by the synthetic resin tube, a stainless steel wire or the like may be embedded.

Although not particularly limited, the outer tube 2 is constituted by a synthetic resin tube such as polyurethane, polyvinyl chloride, polyethylene terephthalate, and polyamide, and a stainless steel wire or the like may be embedded. The inner diameter and the thickness of the outer tube 2 are not particularly limited, and the inner diameter is preferably 1.5 to 4.0 mm, and the thickness is preferably 0.05 to 0.4 mm. A length of the outer tube 2 is preferably 300 to 800 mm.

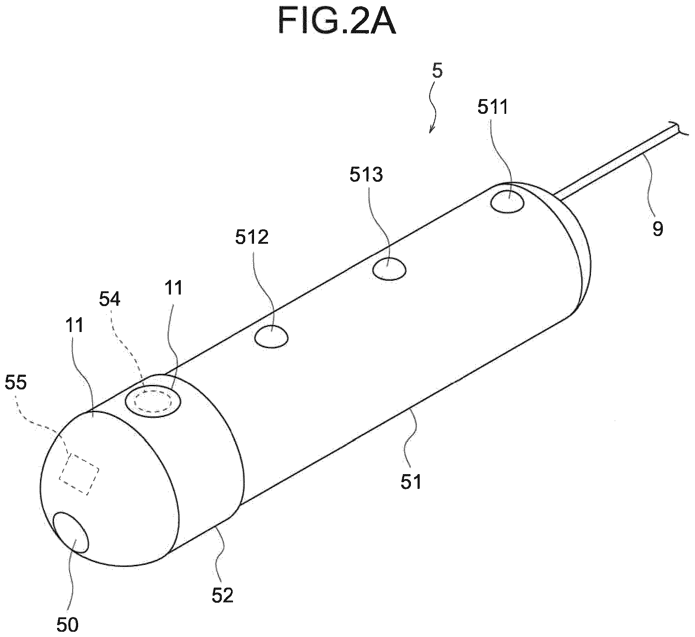

As illustrated in Fig. 2A, the distal tip 5 is roughly consisting of a body portion 51 and a tip end portion 52. The body portion 51 has an external shape of an approximately columnar shape, and constitutes the major part of the distal tip 5. A length of the body portion 51 along the axial direction is longer than a length of the tip end portion 52 along the axial direction. The tip end portion 52 is located on a further distal side in comparison to the body portion 51, and protrudes from a distal end of the body portion 51 to the distal side along the axial direction.

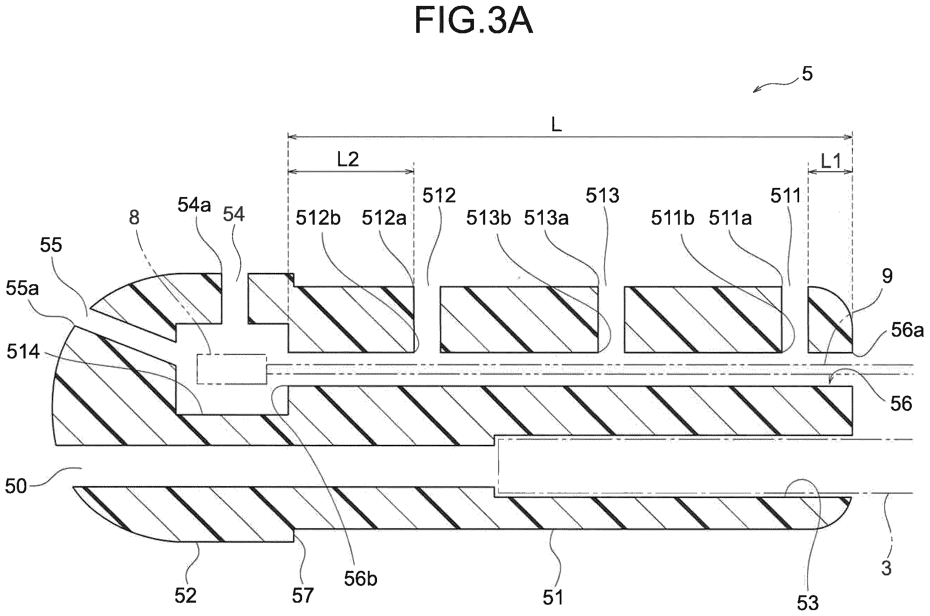

As illustrated in Fig. 3A, the body portion 51 and the tip end portion 52 are integrated, and a step portion 57 is formed at a boundary between the body portion 51 and the tip end portion 52. In the tip end portion 52 located on a further distal side in comparison to the step portion 57, an outer diameter is larger in comparison to the body portion 51 located on a further proximal side in comparison to the step portion 57.

As illustrated in Fig. 1 and Fig. 4C, an outer peripheral surface of the tip end portion 52 is covered with a resin film 11. More specifically, in the outer peripheral surface of the tip end portion 52, a curved surface located on the distal side and a peripheral portion of an opening portion 54a of a lateral side insertion hole 54 are covered with the resin film 11. The resin film 11 is formed on the outer peripheral surface of the tip end portion 52 so that the opening portion 54a of the lateral side insertion hole 54 and an opening portion 55a of a distal side insertion hole 55 are closed. However, at the curved surface of the tip end portion 52, the periphery of an opening portion of a wire insertion hole 50 is not covered with the resin film 11 in order for a guide wire (not illustrated) to be inserted. The resin film 11 is constituted by a material such as urethane resin, a silicone resin, and a polyamide elastomer from the viewpoint of sufficiently securing compatibility with a living body. Note that, in this embodiment, the outer peripheral surface of the tip end portion 52 is locally covered with the resin film 11, but the outer peripheral surface may be entirely covered.

As illustrated in Fig. 3A, an inner tube insertion hole 53 into which the inner tube 3 is inserted is formed in the body portion 51. The inner tube insertion hole 53 extends from a proximal end of the body portion 51 toward the distal side, and a distal end of the inner tube insertion hole 53 is connected to a proximal end of the wire insertion hole 50. The inner tube insertion hole 53 is disposed coaxially and communicates with the wire insertion hole 50, and has a diameter slightly larger (larger by a dimension corresponding to the thickness of the inner tube 3) than a diameter of the wire insertion hole 50. Note that, although details are omitted in the drawings, when the inner tube 3 is inserted into the inner tube insertion hole 53, a distal end of the wire passage 30 of the inner tube 3 is connected to the proximal end of the wire insertion hole 50.

A sensor accommodating hole 514 having an approximately circular column shape is formed inside the tip end portion 52. The sensor accommodating hole 514 is formed in parallel to an axial direction (longitudinal direction) of the distal tip 5, and is a space for accommodating a pressure sensor 8 to be described later. The sensor accommodating hole 514 is disposed on the tip end portion 52 side in the distal tip 5, but may be disposed on the body portion 51 side. In addition, the sensor accommodating hole 514 may be disposed over the body portion 51 and the tip end portion 52.

The lateral side insertion hole 54, the distal side insertion hole 55, and a through-hole 56 are connected to the sensor accommodating hole 514. The lateral side insertion hole 54 is formed so that one end is opened from an outer peripheral surface of the distal tip 5, and the other end is connected to a lateral side (an upward side in the drawing) of the sensor accommodating hole 514.

The lateral side insertion hole 54 extends to a lateral side along a diameter direction of the distal tip 5, and is opened from an outer peripheral surface (non-curved surface) located on the proximal side of the tip end portion 52. The lateral side insertion hole 54 communicates with an inner space of the sensor accommodating hole 514 and the outside of the distal tip 5.

The distal side insertion hole 55 is formed so that one end is opened from the outer peripheral surface (distal end) of the distal tip 5, and the other end is connected to a distal side of the sensor accommodating hole 514.

The distal side insertion hole 55 extends toward the distal side of the distal tip 5 while being inclined to a lateral side (upward side in the drawing), and is opened from an outer peripheral surface (curved surface) located on the distal side of the tip end portion 52. The extension direction of the distal side insertion hole 55 is different from the extension direction of the lateral side insertion hole 54, and an inclination angle of the distal side insertion hole 55 is set, for example, in a range equal to or more than 0° and less than 90° with respect to the axial direction of the distal tip 5. The distal side insertion hole 55 communicates with the inner space of the sensor accommodating hole 514 and the outside of the distal tip 5.

The through-hole 56 is formed so that one end is opened from a proximal end of the distal tip 5, and the other end is connected to a proximal side of the sensor accommodating hole 514. The through-hole 56 extends along the axial direction of the distal tip 5 (the body portion 51). A proximal side opening 56a is formed in a proximal end of the through-hole 56, and a distal side opening 56b is formed in a distal end. The through-hole 56 communicates with the outside of the distal tip 5 and the inner space of the sensor accommodating hole 514 through the proximal side opening 56a and the distal side opening 56b. The optical fiber 9 connected to the pressure sensor 8 to be described later can be inserted into the through-hole 56.

As to be described later, opening widths of the proximal side opening 56a and the distal side opening 56b are set so that the pressure sensor 8 can be inserted when inserting the pressure sensor 8 to which the optical fiber 9 is connected from the outside of the distal tip 5 to the inside of the sensor accommodating hole 514 through the through-hole 56. That is, the opening widths of the proximal side opening 56a and the distal side opening 56b are larger than the maximum width (maximum diameter) of the pressure sensor 8.

A filling-hole (open hole) in which one end is opened from the outer peripheral surface of the distal tip 5 and the other end is connected to the through-hole 56 is formed in the distal tip 5. **In** this embodiment, a plurality of the above-described filling-holes are formed in the distal tip 5. More specifically, three filling-holes including a first filling-hole 511, a second filling-hole 512, and a third filling-hole 513 are arranged in the distal tip 5 along an axial direction of the through-hole 56. The plurality of filling-holes 511 to 513 have an approximately circular column shape, and are connected to the through-hole 56 with constant intervals along the axial direction.

The filling-holes 511 to 513 extend straightly along the diameter direction of the **distal** tip 5 (the body portion 51), and is approximately orthogonal to the through-hole 56. The filling-holes 511 to 513 communicate with an outer space of the distal tip 5 and an inner space of the through-hole 56.

The first filling-hole 511 is connected to a proximal end portion of the through-hole 56. More specifically, the first filling-hole 511 is connected to the through-hole 56 at a position spaced apart from the proximal end of the through-hole 56 to the distal side by a distance L1. When a length of the through-hole 56 is set as L, a relationship of 0 ≤ L1 ≤ L/4 is preferably satisfied.

The second filling-hole 512 is connected to a distal end portion of the through-hole 56. More specifically, the second filling-hole 512 is connected to the through-hole 56 at a position spaced apart from a distal end of the through-hole 56 to the proximal side by a distance L2. When the length of the through-hole 56 is set as L, a relationship of 0 < L2 ≤ L/4 is preferably satisfied.

The third filling-hole 513 is connected to a region between the first filling-hole 511 and the second filling-hole 512 in the through-hole 56. Preferably, the third filling-hole 513 is connected to the through-hole 56 at an equal distance position from the first filling-hole 511 and the second filling-hole 512.

The filling-holes 511 to 513 respectively include first opening portions 511a to 513a opened from the outer peripheral surface of the distal tip 5 (the body portion 51), and second opening portions 511b to 513b opened from an inner wall surface located on a lateral side of the through-hole 56.

The first opening portions 511a to 513a are linearly arranged on the outer peripheral surface of the distal tip 5 (the body portion 51) which is located on a further proximal side in comparison to the sensor accommodating hole 514 along the axial direction. The second opening portions 511b to 513b are linearly arranged on an inner wall surface of the through-hole 56 along the axial direction. The opening portions 511a to 513a, and 511b to 513b of the filling-holes 511 to 513 have approximately circular shape, and the diameter thereof is preferably 0.1 to 0.5 mm.

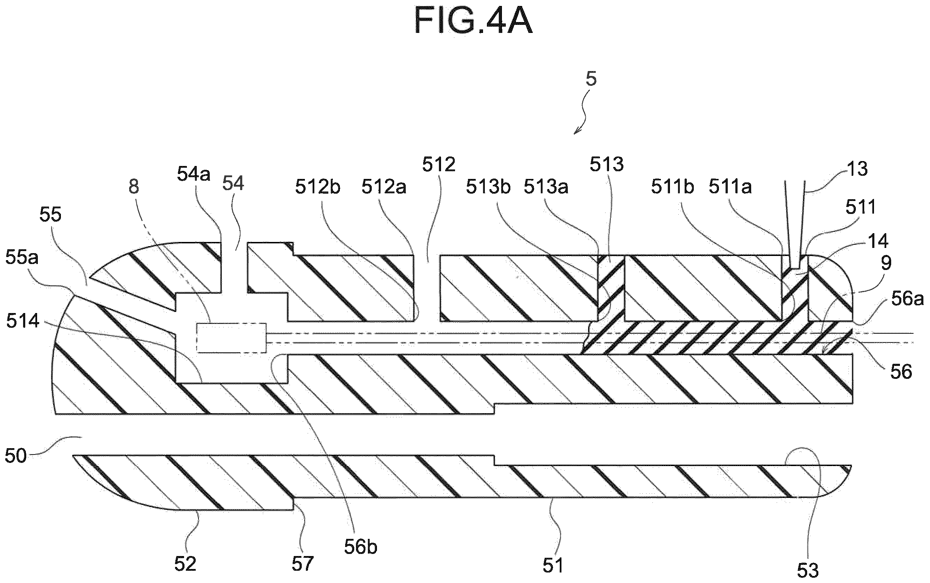

Although details will be described later, in this embodiment, the inside of the through-hole 56 can be filled with the resin 14 through the filling-hole 511 or 513 as illustrated in Fig. 4A to Fig. 4C. In a case where the inside of the through-hole 56 is filled with a sufficient amount of resin 14, the resin 14 is filled up to the vicinity of the first opening portions 511a to 513a of the filling-holes 511 to 513 as illustrated in Fig. 4C.

Although not particularly limited, as the resin 14, a curable resin (adhesive) that has fluidity at the time of filling and is cured after the filling is preferably used. Specific examples of the resin that is used include moisture-curable adhesive such as cyanoacrylate-based adhesive, heat-curable adhesive such as epoxy-based one-component adhesive, and two-component mixed curable adhesive such as epoxy-based two-component adhesive.

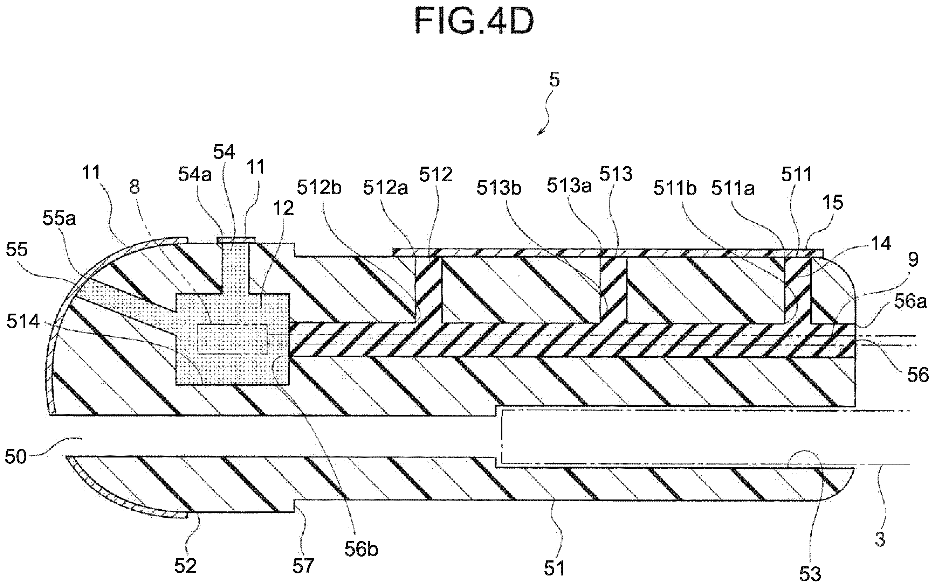

As illustrated in Fig. 4D, a resin film 15 is formed on an outer peripheral surface of the body portion 51. The resin film 15 is formed from the same material of the thin film that constitutes the balloon portion 4 illustrated in Fig. 1, and is formed at a peripheral portion of at least each of the first opening portions 511a to 513a. In this embodiment, the resin film 15 covers the first opening portions 511a to 513a in a closing manner, and the resin 14 located on opening surfaces of the first opening portions 511a to 513a is not exposed to the outside of the distal tip 5 and is covered with the resin film 15.

The resin film 15 continuously cover a region ranging from a distal side of the first opening portion 512a to a proximal side of the first opening portion 511a in the outer peripheral surface of the body portion 51. A width in a direction orthogonal to a longitudinal direction of the resin film 15 is set to a width enough to close the first opening portions 511a to 513a, and is preferably larger than a diameter of each of the first opening portions 511a to 513a.

Although details are omitted in the drawings, a distal end portion 40a of the balloon portion 4 illustrated in Fig. 1 is connected to the outer periphery of the body portion 51 through the resin film 15 at a peripheral portion of each of the first opening portions 511a to 513a, and is not in contact with the resin 14 located on the opening surface of each of the first opening portions 511a to 513a. As described above, the resin film 15 is formed from the same material of the thin film that constitutes the balloon portion 4, and the resin 14 is formed from a material different from the material of the thin film. Accordingly, the resin film 15 is more compatible with the thin film that constitutes the balloon portion 4 in comparison to the resin 14, the distal end portion 40a is connected to the outer periphery (the resin film 15) of the body portion 51 after covering the resin 14 located on the opening surface of each of the first opening portions 511a to 513a with the resin film 15, and thus connection strength or connection reliability therebetween can be enhanced.

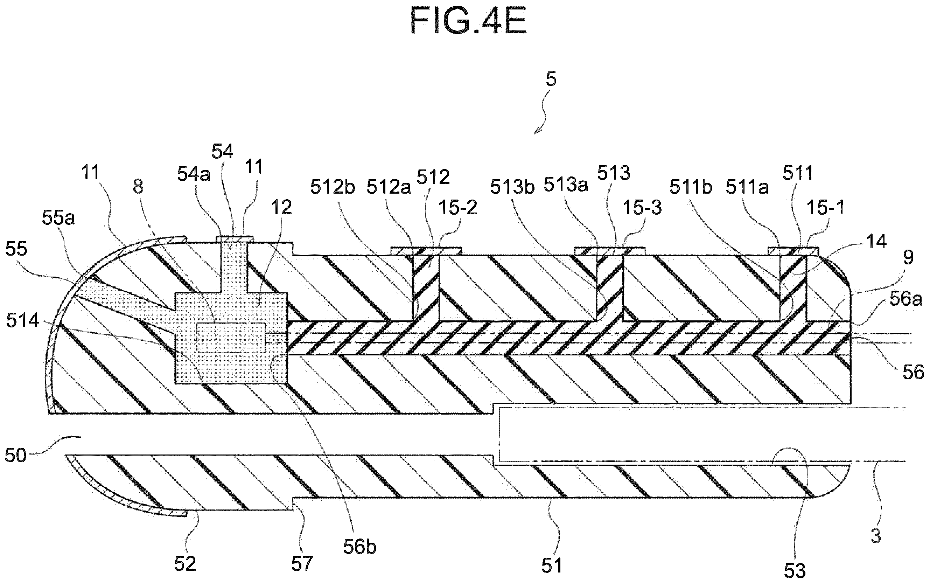

Note that, the resin film 15 may discontinuously cover a region ranging from the distal side of the first opening portion 512a to the proximal side of the first opening portion 511a in the outer peripheral surface of the body portion 51. For example, as illustrated in Fig. 4E, only a peripheral portion of each of the first opening portions 511a to 513a in the outer peripheral surface of the body portion 511 may be locally covered with each of three resin films 15_1 to 15_3.

The pressure sensor 8 is a sensor that detects a pressure (blood pressure) in a space inside the sensor accommodating hole 514 by using a path difference of light transmitted through the optical fiber 9. As the pressure sensor 8, a pressure sensor described in JP 2008-524606 A, JP 2000-35369 A, or the like can be used.

In the sensor accommodating hole 514, for example, a space around the pressure sensor 8 is filled with a pressure transfer filler such as a gel-like substance 12 (refer to Fig. 4C) such as silicone gel, polyacrylamide gel, and polyethylene oxide gel, and an oil-like substance such as silicone oil.

Next, description will be given of a method of manufacturing the intra-aortic balloon catheter 1 of the invention with focus given to a method of fixing the optical fiber 9 in which the pressure sensor 8 is connected to the distal end thereof to the inside of the through-hole 56 of the distal tip 5 with reference to the accompanying drawings.

First, the distal tip 5, and the pressure sensor 8 to which the distal end of the optical fiber 9 is connected are prepared, and the pressure sensor 8 is inserted into the through-hole 56 from the proximal side opening portion 56a and is pushed toward the distal side through the through-hole 56 until being disposed inside the sensor accommodating hole 514. According to this, as illustrated in Fig. 4A, the pressure sensor 8 is accommodated inside the sensor accommodating hole, and a distal end portion of the optical fiber is disposed inside the through-hole 56. A formation method of the distal tip 5 is not particularly limited, and may be manufactured by using a synthetic resin material such as polyurethane, polyvinyl chloride, polyethylene terephthalate, and a polyamide, or various kinds of metal materials such as an Ni-Ti alloy, for example, by an injection molding method.

Next, a syringe 13 filled with the curable resin 14 is inserted into the first filling-hole 511, the resin 14 is discharged from the syringe 13, and the resin 14 is filled (poured) into the through-hole 56 through the first filling-hole 511.

After initiation of the filling, the resin 14 flows from the second opening portion 511b of the first filling-hole 511 into the through-hole 56, and flows toward a proximal side and a distal side of the through-hole 56. When the resin 14 flowing to the distal side is filled into the through-hole 56 in a region between the first filling-hole 511 and the third filling-hole 513 without a gap (sufficiently), the resin 14 flows from the through-hole 56 to the third filling-hole 513, and is filled up to the vicinity of the first opening portion 513a.

Accordingly, it is possible to understand a filling state of the resin 14 inside the through-hole 56 in the region between the first filling-hole 511 and the third filling-hole 513 by confirming a filling state of the resin 14 filled inside the third filling-hole 513. In addition, in correspondence with the filling state of the resin 14 inside the through-hole 56 in the region, a filling amount of the resin 14 can be adjusted, and the inside of the through-hole 56 in the region can be filled with an optimal amount of resin.

Note that, it is possible to understand the filling state of the resin 14 inside the through-hole 56 in a region on a further proximal side in comparison to the first filling-hole 511 by confirming whether or not the resin 14 approaches the proximal side opening portion 56a of the through-hole 56.

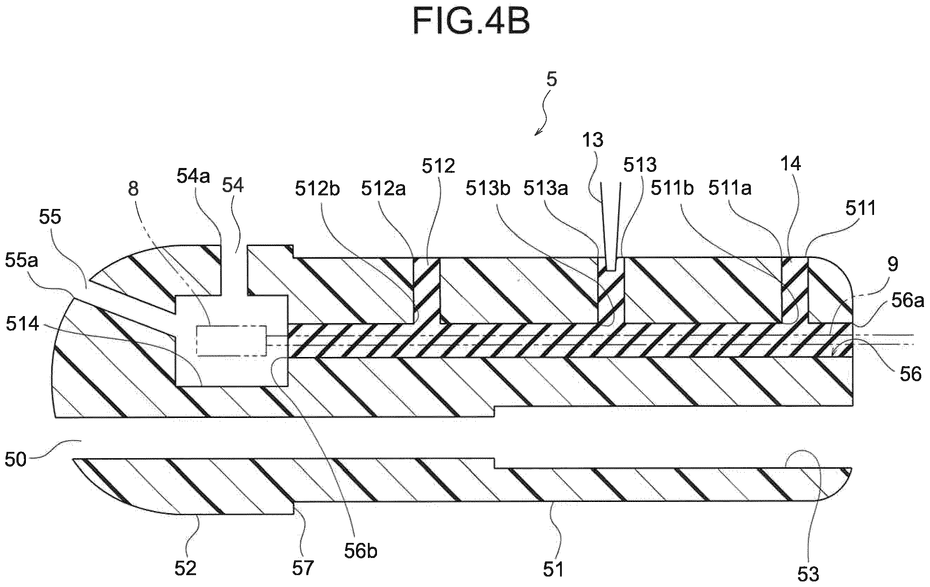

Next, as illustrated in Fig. 4B, the syringe 13 is pulled out from the first filling-hole 511, and the syringe 13 is inserted into the third filling-hole 513. Then, the resin 14 is discharged from the syringe 13, and the resin 14 is filled (poured) into the through-hole 56 through the third filling-hole 513.

After initiation of the filling, the resin 14 flows from the second opening portion 513b of the third filling-hole 513 into the through-hole 56, and flows toward the distal side of the through-hole 56. When the resin 14 flowing to the distal side is filled into the through-hole 56 in a region between the second filling-hole 512 and the third filling-hole 513 without a gap (sufficiently), the resin 14 flows from the through-hole 56 to the second filling-hole 512, and is filled up to the vicinity of the first opening portion 512a.

Accordingly, it is possible to understand a filling state of the resin 14 inside the through-hole 56 in the region between the second filling-hole 512 and the third filling-hole 513 by confirming a filling state of the resin 14 filled inside the second filling-hole 512.

In addition, since the inside of the through-hole 56 is filled with the resin 14 through two filling-holes (at least one resin injection hole) 511 and 513, and the filling state of the resin 14 in the through-hole 56 can be confirmed through the two filling-holes (at least one filling confirmation hole) 512 and 513, the amount of the resin 14 filled in the through-hole 56 can be finely adjusted. In addition, the inside of the through-hole 56 and the filling-holes 511 to 513 are filled with the resin (a sufficient amount of resin is supplied into the through-hole 56), and thus the fixing strength of the optical fiber 9 fixed to the inside of the through-hole 56 can be sufficiently enhanced.

Note that, the resin 14 may be discharged from the syringe 13 simultaneously with insertion of the syringe 13 into the second filling-hole 512, and the resin 14 may be filled (supplemented) into the through-hole 56 in a region on a further distal side in comparison to the second filling-hole 512 through the second filling-hole 512 as necessary.

Next, as illustrated in Fig. 4C, the inside of the sensor accommodating hole 514 is filled with the gel-like substance 12 through the lateral side insertion hole 54. According to this, the inside of the sensor accommodating hole 514, the lateral side insertion hole 54, and the distal side insertion hole 55 is filled with the gel-like substance 12, and thus the pressure sensor 8 accommodated in the sensor accommodating hole 514 is covered (fixed) with the gel-like substance 12. In this embodiment, since the sensor accommodating hole 514 communicates with the outside of the distal tip 5 through the lateral side insertion hole 54 and distal side insertion hole 55, air bubbles are likely to be leaked from the distal side insertion hole 55 at the time of filling of the gel-like substance 12, and thus air bubbles can be prevented from remaining the inner space of the sensor accommodating hole 514.

In this embodiment, the outside of the distal tip 5 where a blood pressure is to be measured and the pressure sensor 8 enter a communicating state through the lateral side insertion hole 54 and the distal side insertion hole 55, and a pressure near the distal tip 5 is detected by the pressure sensor 8. In addition, as illustrated in Fig. 4C, in a case where the inside of the sensor accommodating hole 514 is filled with the pressure transfer filler (the gel-like substance 12), the pressure of the outside of the distal tip 5 where a blood pressure is to be measured is transferred through the pressure transfer filler, and this pressure is detected by the pressure sensor 8.

Next, the resin film 11 is formed on the outer peripheral surface (non-curved surface) of the proximal side of the tip end portion 52 of the distal tip 5, for example, in an approximately circular shape to close the opening portion 54a of the lateral side insertion hole 54. In addition, the resin film 11 is formed on the outer peripheral surface (curved surface) on the distal side of the tip end portion 52 of the distal tip 5 to close the opening portion 55a on the distal side insertion hole 55. According to this, the gel-like substance 12 filled in the sensor accommodating hole 514 is prevented from being leaked to the outside of the distal tip 5. Note that, the opening portion on the distal side of the wire insertion hole 50 is not closed with the resin film 11 and is opened.

In addition, as illustrated in Fig. 4D, the resin film 15 is formed on the outer peripheral surface of the body portion 51 to close the first opening portions 511a to 513a.

Next, a distal side of the inner tube 3 is inserted into the inner tube insertion hole 53 of the body portion 51 to be connected and fixed thereto, and the distal end portion 40a (refer to Fig. 1) of the balloon portion 4 is fixed to the outer peripheral surface on a proximal side of the body portion 51. According to this, the intra-aortic balloon catheter 1 illustrated in Fig. 1 is manufactured. Note that, in the outer peripheral surface of the body portion 51, with regard to a portion on which the resin film 15 is formed, the distal end portion 40a is fixed to the surface of the resin film 15.

In the intra-aortic balloon catheter 1 according to this embodiment, the filling-holes 511 to 513 are formed in the distal tip 5. Accordingly, the inside of the through-hole 56 can be directly filled with the resin 14 through the filling-holes 511 to 513, and thus the optical fiber 9 can be fixed to the inside of the through-hole 56 with the resin 14. That is, in the intra-aortic balloon catheter 1 according to this embodiment, it is not necessary fill the inside of the sensor accommodating hole 514 with the resin 14 unlike the method of fixing an optical fiber illustrated in the related art. According to this, there is no concern that the resin 14 adheres to the pressure sensor 8 disposed in the sensor accommodating hole 514, deterioration of accuracy of the pressure sensor 8 due to adhesion of the resin 14 is prevented, and fluctuations in a blood pressure inside an aorta can be measured with high accuracy.

In addition, since the inside of the through-hole 56 is directly filled with the resin 14, a void is less likely to occur in a resin layer formed in the through-hole 56. Accordingly, the resin 14 is prevented from being peeled off from an inner wall surface of the through-hole 56, and the optical fiber 9 can be fixed to the inside of the through-hole 56 with high fixing strength.

In addition, the filling-holes 511 to 513 extend along a diameter direction of the distal tip 5. According to this, the outer peripheral surface of the distal tip 5 and the through-hole 56 are connected at the shortest distance through the filling-holes 511 to 513, and thus the inside of the through-hole 56 can be easily filled with the resin 14 through the filling-holes 511 to 513.

### Second Embodiment

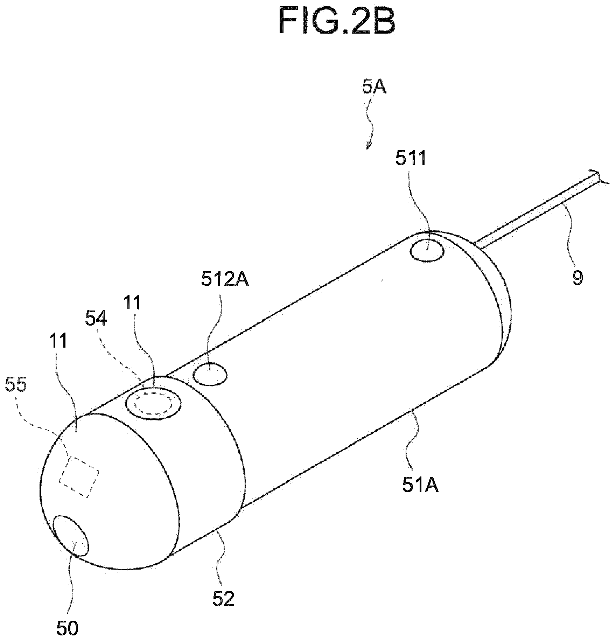

As illustrated in Fig. 2B, an intra-aortic balloon catheter according to a second embodiment of the invention includes a distal tip 5A. The distal tip 5A in this embodiment is different from the distal tip 5 in the first embodiment in that the distal tip 5A includes a body portion 51A. In the following description, description relating to a portion common to the first embodiment will be omitted, and a common reference numeral will be given to a common member.

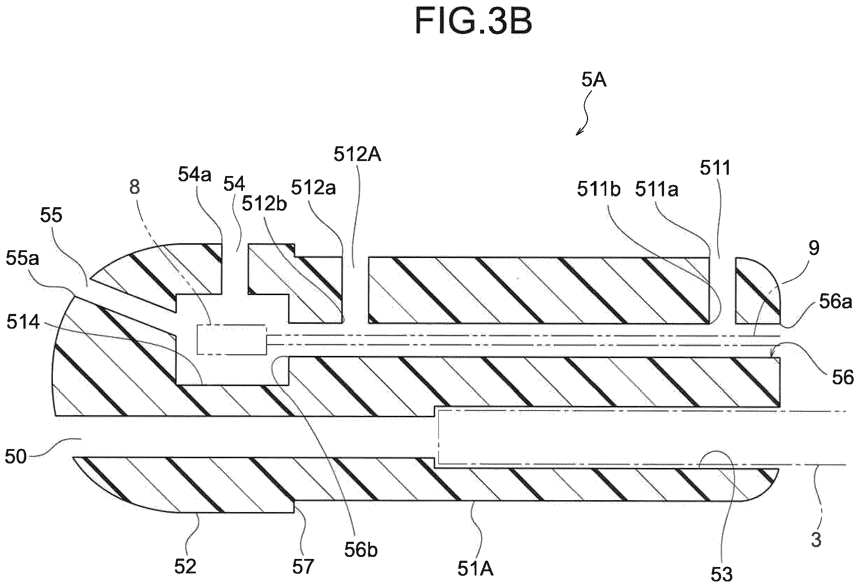

As illustrated in Fig. 2B and Fig. 3B, the body portion 51A is different from the body portion 51 in the first embodiment in that the third filling-hole 513 illustrated in Fig. 2A and Fig. 3A is not provided. In addition, the body portion 51A is different from the body portion 51 in the first embodiment in that a second filling-hole 512A is disposed on a further distal side of the through-hole 56 in comparison to the second filling-hole 512 illustrated in Fig. 3A.

In this embodiment, the inside of the through-hole 56 is filled with the resin 14 through the first filling-hole 511, and a filling state of the resin 14 inside the through-hole 56 in a region between the first filling-hole 511 and the second filling-hole 512A can be confirmed through the second filling-hole 512A. In addition, in this embodiment, the number of the filling-holes formed in the distal tip 5A is less than the number of the filling-holes formed in the distal tip 5 illustrated in Fig. 2A, and thus the configuration of the distal tip 5A can be further simplified in comparison to the first embodiment.

In addition, since the second filling-hole 512A is disposed at the above-described position, a wide region ranging from the proximal end portion to the distal end portion of the through-hole 56 can be easily filled with a sufficient amount of resin 14. Note that, the second filling-hole 512A may be disposed on a further distal side of the through-hole 56 (distal end of the through-hole 56).

Note that, the invention is not limited to the above-described embodiments, and various modifications can be made within the scope of the invention, which is defined by the appended claims.

In addition, in the distal tip 5 illustrated in Fig. 2A, the filling-holes 511 to 513 may be formed on the outer peripheral surface of the body portion 51 to be displaced in a peripheral direction. In addition, the filling-holes 511 to 513 along the axial direction may not be equally spaced.

In addition, in Fig. 3A, the filling-holes 511 to 513 extend straightly along the diameter direction of the distal tip 5, but may extend obliquely. This is also true of the lateral side insertion hole 54.

In addition, for example, the shape of the filling-holes 511 to 513 may be set to an approximately quadrangular prism shape, an approximately triangular prism shape, or the like.

In the above-described first embodiment, the resin film 11 formed on the outer peripheral surface of the tip end portion 52 may be omitted.

In the above-described embodiments, the diameter of the opening portions 511a to 513a, and 511b to 513b is set to be smaller than the diameter of the proximal side opening portion 56a and the distal side opening portion 56b of the through-hole 56, but may be set to be larger than the diameter of the proximal side opening portion 56a and the distal side opening portion 56b of the through-hole 56. With this configuration, the inside of the through-hole 56 is easily filled with the resin 14 without a gap.

### EXPLANATIONS OF LETTERS OR NUMERALS

1 INTRA-AORTIC BALLOON CATHETER
2 OUTER TUBE
   20 PRESSURE FLUID CONDUCTION PATH
3 INNER TUBE
   30 WIRE PASSAGE
4 BALLOON PORTION
   40a DISTAL END PORTION
   40b PROXIMAL END PORTION
5, 5A DISTAL TIP
   50 WIRE INSERTION HOLE
   51, 51A BODY PORTION
      511 FIRST FILLING-HOLE
         511a FIRST OPENING PORTION
         511b SECOND OPENING PORTION
      512, 512A SECOND FILLING-HOLE
         512a FIRST OPENING PORTION
         512b SECOND OPENING PORTION
      513 THIRD FILLING-HOLE
         513a FIRST OPENING PORTION
         513b SECOND OPENING PORTION
      514 SENSOR ACCOMMODATING HOLE
   52 TIP END PORTION
   53 INNER TUBE INSERTION HOLE
   54 LATERAL SIDE INSERTION HOLE
      54aOPENING PORTION
   55 DISTAL SIDE INSERTION HOLE
      55aOPENING PORTION
   56 THROUGH-HOLE
      56aPROXIMAL SIDE OPENING PORTION
      56b DISTAL SIDE OPENING PORTION
   57 STEP PORTION
6 CONTRAST MARKER
7 BRANCH PORTION
   71 PRIMARY PORT
   72 SECONDARY PORT
   73 TERTIARY PORT
   74 PRIMARY PASSAGE
   75 SECONDARY PASSAGE
   76 TERTIARY PASSAGE
8 PRESSURE SENSOR
9 OPTICAL FIBER
10 OPTICAL CONNECTOR
11, 15, 15_1 to 15_3 RESIN FILM
12 GEL-LIKE SUBSTANCE
13 SYRINGE
14 RESIN

## Claims

1. An intra-aortic balloon catheter (1) comprising:
a sensor (8) capable of measuring a pressure by using light;
an optical fiber (9) connected to the sensor (8); and
a distal tip (5) having a sensor accommodating hole (514) accommodating the sensor (8) and a through-hole (56) connected to the sensor accommodating hole (514) to pass the optical fiber (9) through,
wherein filling-holes (511, 512, 513) in which one end is opened from an outer peripheral surface of the distal tip (5) and the other end is connected to the through-hole (56) are formed in the distal tip (5) in a manner capable of filling the inside of the through-hole (56) with a resin (14),
**characterised in that** the inside of the through-hole (56) and the filling-holes (511, 512, 513) is filled with the resin (14)
each of the filling-holes (511, 512, 513) is disposed at the distal tip (5) along an axial direction of the through-hole (56), and
an inner wall of the through-hole (56) is fixed to the optical fiber (9) with the resin (14).

2. The intra-aortic balloon catheter (1) according to claim 1,
wherein a first filling-hole (511) of the filling-holes (511, 512, 513) is connected to a proximal end portion of the through-hole (56), and
a second filling-hole (512) of the filling-holes (511, 512, 513) is connected to a distal end portion of the through-hole (56).

3. The intra-aortic balloon catheter (1) according to claim 2,
wherein a third filling-hole (513) of the filling-holes (511, 512, 513) is connected to the through-hole (56), and
the third filling-hole (513) is located between the first filling-hole (511) and the second filling-hole (512).

4. The intra-aortic balloon catheter (1) according to any one of claims 1 to 3,
wherein the filling-holes (511, 512, 513) extend along a diameter direction of the distal tip (5).

## Patentansprüche

1. Intra-aortaler Ballonkatheter (1) umfassend:
einen Sensor (8), der fähig ist, mit Hilfe von Licht einen Druck zu messen;
eine optische Faser (9), die mit dem Sensor (8) verbunden ist; und
eine distale Spitze (5) mit einem Sensoraufnahmeloch (514), das den Sensor (8) aufnimmt, und einem Durchgangsloch (56), das mit dem Sensoraufnahmeloch (514) verbunden ist, um die optische Faser (9) hindurchzuführen,
wobei Fülllöcher (511, 512, 513), bei denen ein Ende von einer äußeren Umfangsfläche der distalen Spitze (5) aus geöffnet ist und das andere Ende mit dem Durchgangsloch (56) verbunden ist, in der distalen Spitze (5) derart ausgebildet sind, dass das Innere des Durchgangslochs (56) mit einem Harz (14) füllbar ist,
**dadurch gekennzeichnet, dass** das Innere des Durchgangslochs (56) und der Fülllöcher (511, 512, 513) mit dem Harz (14) gefüllt ist,
jedes der Fülllöcher (511, 512, 513) an der distalen Spitze (5) entlang einer axialen Richtung des Durchgangslochs (56) angeordnet ist, und
eine Innenwand des Durchgangslochs (56) mit dem Harz (14) an der optischen Faser (9) befestigt ist.

2. Intra-aortaler Ballonkatheter (1) nach Anspruch 1,
wobei ein erstes Füllloch (511) der Fülllöcher (511, 512, 513) mit einem proximalen Endabschnitt des Durchgangslochs (56) verbunden ist, und
ein zweites Füllloch (512) der Fülllöcher (511, 512, 513) mit einem distalen Endabschnitt des Durchgangslochs (56) verbunden ist.

3. Intra-aortaler Ballonkatheter (1) nach Anspruch 2,
wobei ein drittes Füllloch (513) der Fülllöcher (511, 512, 513) mit dem Durchgangsloch (56) verbunden ist, und
das dritte Füllloch (513) sich zwischen dem ersten Füllloch (511) und dem zweiten Füllloch (512) befindet.

4. Intra-aortaler Ballonkatheter (1) nach einem der Ansprüche 1 bis 3,
wobei sich die Fülllöcher (511, 512, 513) entlang einer Durchmesserrichtung der distalen Spitze (5) erstrecken.

## Revendications

1. Cathéter à ballonnet intra-aortique (1) comprenant :
un capteur (8) capable de mesurer une pression en utilisant de la lumière ;
une fibre optique (9) raccordée au capteur (8) ; et
une pointe distale (5) ayant un trou de logement de capteur (514) logeant le capteur (8) et un trou débouchant (56) raccordé au trou de logement de capteur (514) pour y faire passer la fibre optique (9),
dans lequel des trous de remplissage (511, 512, 513), dans lesquels une extrémité est ouverte à partir d'une surface périphérique externe de la pointe distale (5) et l'autre extrémité est raccordée au trou débouchant (56), sont formés dans la pointe distale (5) de manière à pouvoir remplir l'intérieur du trou débouchant (56) avec une résine (14),
**caractérisé en ce que** :
l'intérieur du trou débouchant (56) et des trous de remplissage (511, 512, 513) est rempli avec de la résine (14),
chacun des trous de remplissage (511, 512, 513) est disposé au niveau de la pointe distale (5) le long d'une direction axiale du trou débouchant (56), et
une paroi interne du trou débouchant (56) est fixée à la fibre optique (9) avec la résine (14).

2. Cathéter à ballonnet intra-aortique (1) selon la revendication 1,
dans lequel un premier trou de remplissage (511) des trous de remplissage (511, 512, 513) est raccordé à une partie d'extrémité proximale du trou débouchant (56), et
un deuxième trou de remplissage (512) des trous de remplissage (511, 512, 513) est raccordé à une partie d'extrémité distale du trou débouchant (56).

3. Cathéter à ballonnet intra-aortique (1) selon la revendication 2,
dans lequel un troisième trou de remplissage (513) des trous de remplissage (511, 512, 513) est raccordé au trou débouchant (56), et
le troisième trou de remplissage (513) est situé entre le premier trou de remplissage (511) et le deuxième trou de remplissage (512).

4. Cathéter à ballonnet intra-aortique (1) selon l'une quelconque des revendications 1 à 3,
dans lequel les trous de remplissage (511, 512, 513) s'étendent le long d'une direction diamétrale de la pointe distale (5).
